# EUROPEAN PATENT SPECIFICATION

(11) **EP 2 403 565 B1**
(45) Date of publication and mention of the grant of the patent: **01.01.2014**
(21) Application number: 10709281.9
(22) Date of filing: 25.02.2010
(51) Int. Cl.: A61M 5/315

(54) **PRESSURIZED RESERVOIR SYSTEM FOR STORING AND DISPENSING LIQUIDS**
UNTER DRUCK STEHENDES RESERVOIRSYSTEM ZUR AUFBEWAHRUNG UND ABGABE VON FLÜSSIGKEITEN
SYSTÈME DE RÉSERVOIR SOUS PRESSION POUR LE STOCKAGE ET LA DISTRIBUTION DE LIQUIDES

(30) Priority: 26.02.2009 US 155530 P
(43) Date of publication of application: 11.01.2012
(73) Proprietor: G-Sense Ltd., 39032 Tirat Carmel (IL)
(72) Inventor: SHEKALIM, Avraham, 36842 Nesher (IL); PELEG, Noam, 19351 Gan-Ner (IL)
(74) Representative: Watterson, Peer Marten John
(86) International application number: PCT/IB2010/050833
(87) International publication number: WO 2010/097774

(56) References cited:
- WO-A1-86/06967
- WO-A2-2006/118949
- FR-A1- 2 799 654
- US-A- 5 704 918

## Description

### FIELD AND BACKGROUND OF THE INVENTION

The present invention generally relates to a pressurized reservoir system for storing and dispensing a plurality of liquids in very small quantities and, particularly, is concerned with two separate liquid dispensing schemes. The first dispensing scheme involves dispensing the liquids independently from each other and the second scheme involves dispensing them together in fixed volumetric ratios.

The difficulty precision dispensing of liquids in miniaturized liquid delivery systems is well-known. Some situations require liquids to be delivered independently while other situations require them to be delivered simultaneously in fixed volumetric proportions. PCT publication WO 2008/056363 discloses a particularly efficient delivery system for delivery of a plurality of liquids simultaneously in fixed volumetric proportion. However, it does not teach a method or system for delivering a plurality of liquids independently. Therefore, there is a need for a liquid delivery system capable of dispensing a plurality of liquids on an independent basis and also simultaneously in fixed volumetric proportion.

### SUMMARY OF THE INVENTION

The present invention is a pressurized reservoir system for storing and dispensing liquids.

According to the teachings of the present invention there is provided, a pressurized reservoir system for storing and dispensing liquids comprising: (a) a housing, (b) a piston arrangement in the housing including at least one piston, the piston arrangement at least partially defining at least two liquid-storage volumes not in fluid communication with each other, each of the liquid-storage volumes having a flow path for dispensing a stored liquid, and (c) a resilient biasing element configured to bias the piston arrangment to pressurize the liquid-storage volumes, wherein the piston arrangement is configured to independently dispense each of the liquids stored in the liquid-storage volumes.

According to a further feature of the present invention, the piston arrangement comprises two pistons in axial alignment, the biasing element being disposed between the two pistons so as to bias both of the two pistons in opposite directions.

According to a further feature of the present invention, the piston arrangement further comprises at least one floating.

According to a further feature of the present invention, the piston arrangement comprises at least one piston having a cavity of parallel walls, wherein the parallel walls slidingly engage an extended body thereby at least partially defining a first of the two liquid-storage volumes.

According to a further feature of the present invention, the at least one piston has an external surface extending from the parallel walls so as to be in sliding engagement with the housing thereby partially defining a second of the two liquid-storage volumes not in fluid communication with each other.

According to a further feature of the present invention, the at least one piston is implemented as a floating piston.

According to a further feature of the present invention, the extended body includes a static projection from the housing.

According to a further feature of the present invention, the static projection includes flow path of the first liquid-storage.

According to a further feature of the present invention, the housing comprises a cylindrical wall.

According to a further feature of the present invention, the biasing element comprises a spring.

According to a further feature of the present invention, the biasing element comprises a compressed gas.

According to a further feature of the present invention, there is also provided a valve-actuator control-system configured for controlling valves regulating flow in the flow paths.

According to a further feature of the present invention, there is also provided an outlet conduit at least partially containing one of the flow paths, the outlet conduit passing through an opening in the primary piston and extending into one of the liquid-storage volumes, the primary piston being in sealed sliding engagement with the outlet conduit.
WO 86/06967 A1 discloses an automatic injector apparatus in which two ingredients are separately stored and are then mixed prior to dispensing, for subsequent dispensing of the combined mixture.
WO 2006/118949 A2 discloses a two-chambered, sequentially injectable syringe.
FR 2 799 654 A1 discloses a syringe-type device for freshly preparing a solution, suspension or dispersion, for example injectable formulations, preferably according to a method for reconstitution in a vacuum.
US 5 704 918 A discloses a syringe, in which two injection agents can be sequentially injected separately or mixedly by a simple operation.

### BRIEF DESCRIPTION OF THE DRAWINGS

The invention is herein described, by way of example only, with reference to the accompanying drawings, wherein:
FIG. 1 is an isometric, cross-sectional side view of a reservoir system depicting a plurality of reservoirs in a pre-dispensing state.
FIG. 2 is an isometric, cross-sectional side view of the reservoir system after independent dispensing state of one liquid, but prior to linked-dispensing of liquids remaining in the reservoir system.
FIG. 3 is an isometric, cross-sectional side view of an alternative embodiment of the reservoir system including a control system depicting the system before a biasing spring has not been loaded.
FIG. 4 is an isometric, cross-sectional side view of the reservoir system of FIG. 1 pre-dispensing state with the biasing spring loaded and reservoirs filled.
FIGS. 5-7 are schematic, cross-sectional side-views of an alternative embodiment of a reservoir system employing a common biasing spring disposed between two pistons at pre-dispensing, post-independent, intermediate and post-dispensing stages, respectively.

### DESCRIPTION OF THE PREFERRED EMBODIMENTS

An embodiment of the present invention is a miniaturized reservoir system for storing and dispensing very small amounts of liquid in a combination dispensing scheme. The system dispenses at least two liquids independently from each other and, optionally, also dispenses liquids in predefined, fixed volumetric-ratios.

The principles and operation of miniaturized reservoir system according to the present invention may be better understood with reference to the drawings and the accompanying description.

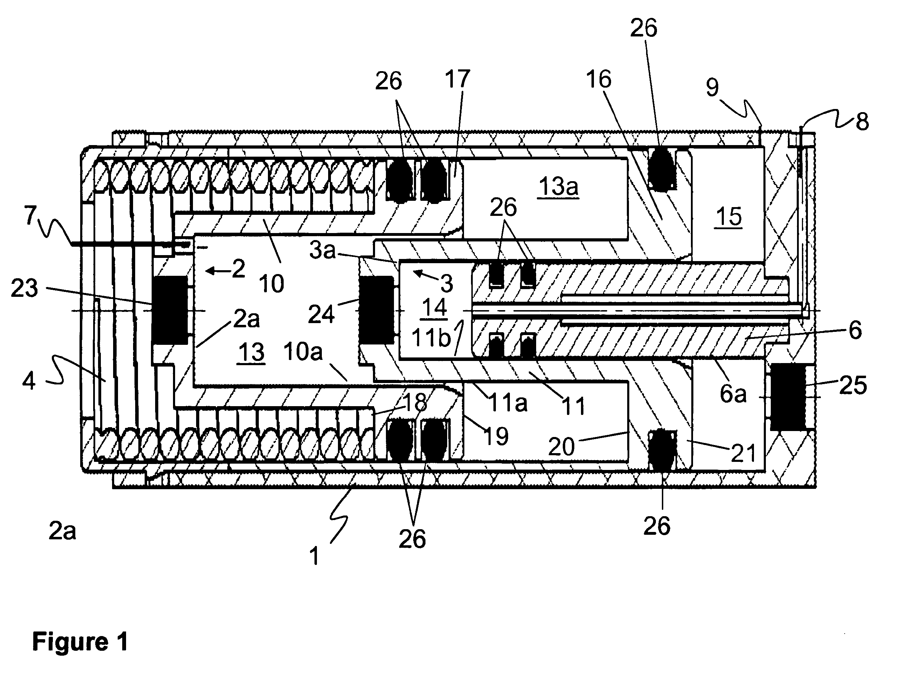

Referring now to the drawings, Figure 1 depicts a non-limiting, exemplary embodiment of a reservoir system in an initial pre-dispensing state in which each reservoir is fully supplied with an appropriate liquid. It should be noted that for the sake of convenience the present document discusses the reservoir system in the context of a combined insulin infusion and glucose monitor based on a microdialysis probe arrangement; however, it should be appreciated that any system requiring storage and precise delivery of a plurality of liquids is included within the scope of the present invention. The reservoir system includes a system housing **1,** a primary piston **2** in axial alignment with a secondary piston **3** forming an overall piston arrangement, a spring **4** for biasing the piston arrangement to advance in housing **1,** a static projection **6,** a primary flow path **7,** a secondary flow path **8** which extends in part along static projection **6,** and a tertiary flow path **9.** Primary piston **2** includes a parallel piston wall **10,** typically of cylindrical form and terminating in a closure **2a,** that forms a primary cavity **13.** Similarly, secondary piston **3** includes a parallel piston wall **11,** typically of cylindrical form and terminating in a closure **3a,** forming a secondary cavity **14.** The diameter of a cylindrical external surface **11*a*** of wall **11** is less than the diameter of a cylindrical internal surface **10*a*** of wall **10** so that primary and secondary pistons **2** and **3** can nest. Optionally, the two cylindrical walls may be configured to engage in sliding engagement, but preferably with flow channels formed in one or both surfaces to interconnect volumes **13** and **13*a*.** Alternatively, there may be a clearance there between. Static projection **6** is parallel walled, and preferably cylindrical, with an outer surface **6*a*** configured to engage and seal against the inner surface **11*b*** of wall **11,** thereby isolating storage volumes **14** and **15** from each other. Primary cavity **13** and secondary cavity **14** each serve as liquid storage volumes not in fluid communication with each other because they are divided by floating, secondary piston **3** thereby enabling liquids to be dispensed from primary storage volume **13** while liquids in secondary storage volume **14** remain in storage as will be further discussed. The above-described piston arrangement is in axial alignment with spring **4** disposed at one end of housing **1.**

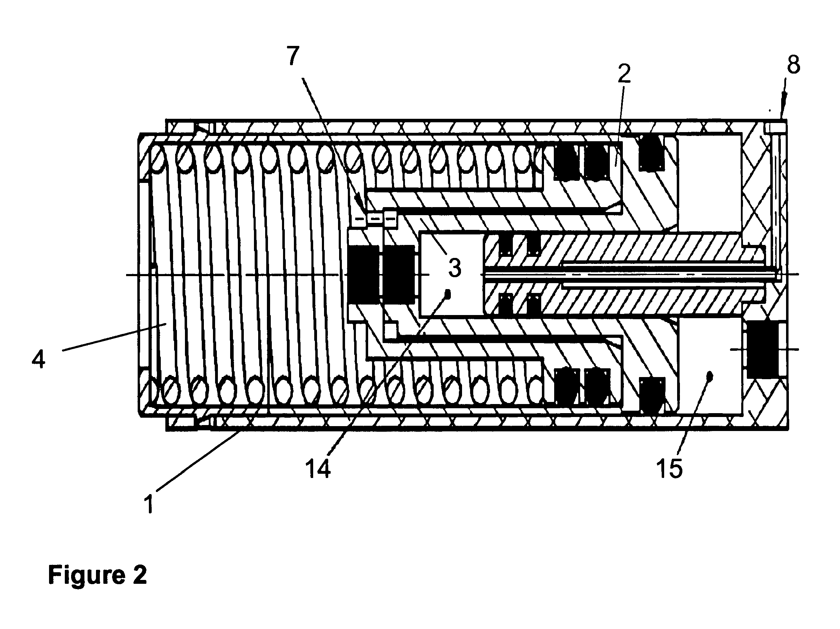

At this point it is helpful to define some terms of reference used throughout the present document.
- "Fluid Communication" refers to a flow arrangement between a plurality of liquid storage volumes in which liquid flows from one storage volume to another.
- "Parallel Walls" refers to any wall arrangement in which the distance between opposing walls or opposing sections of a single wall is constant.
- "Floating Piston" refers to a piston not acted upon by a mechanical linkage but rather is acted upon by liquids in which the piston is disposed.
- "Distal" refers to a side most distant from a biasing element.
- "Flange" refers to any surface extending outwards from the piston wall towards the housing.
Primary flange **17** is disposed at a distal end of the primary piston wall **10** and primary flange **17** extends radially to housing **1** where it slidingly engages it. Similarly, secondary flange **16** is disposed at the distal end of piston wall **11** and extends radially to housing **1** where it also slidingly engages it. Seals **26,** preferably implemented as "o-rings", are disposed in between flange surfaces in sliding contact with housing **1** and walls **6a** of static projection **6** to ensure a leak-free sliding engagement. Distal surface **19** of primary flange **17** and non-distal surface **20** of secondary flange **16** partially define an additional liquid storage volume **13a** that in the present non-limiting embodiment is in fluid communication with primary storage volume **13.** Distal surface **21** of secondary flange **16** partially defines tertiary liquid storage volume **15.** In a non-limiting exemplary embodiment, primary flow path **7** (shown here schematically) is in fluid connection with primary storage volume **13** and passes through the space circumscribed by spring **4** as shown in Figures 1 and 2. Secondary flow path **8** is fluid communication with secondary liquid storage volume **14** and passes through static projection **6** as mentioned above. Tertiary flow path **9** is in fluid communication with tertiary liquid storage volume **15.** As mentioned above, spring **4** is disposed at one end of housing **1** and applies a bias to the piston arrangement. In this particular, non-limiting embodiment, spring **4** resiliently bears directly on non-distal surface **19** of primary flange **2** thereby pressurizing liquids in volume **13**, and indirectly, by the liquid pressure acting on surfaces of the floating secondary piston **3,** also pressurizes liquids in secondary and tertiary volumes **14** and **15,** respectively. Each flow path typically includes a valve and valve actuator (not shown) controlled by a control system in accordance with system parameters; closed valves maintain the liquids in liquid-storage volumes and open valves dispense the liquids as is known in the art. It should be noted that, in a non-limiting, exemplary embodiment, the housing is implemented as a cylindrical wall. However, non-cylindrical embodiments are included within the scope of the present invention.

Figure 2 depicts the reservoir system after dispensing of the liquid stored in the combined storage volumes **13** and **13*a*,** but where the liquids in storage volumes **14** and **15** have not yet been dispensed. It will be noted that this state has been chosen for clarity of presentation, but there is no limitation as to the sequence and relative rates of dispensing of the liquid in storage volume **13, 13*a*** versus that in storage volume **14.** Independent dispensing commences when a valve in flow path **7** is opened. Primary piston **2,** biased by spring **4,** advances in housing **1** and expels liquids stored liquid-storage volume **13** as without affecting liquids stored in other storage volumes **14** or **15.** This independent type of liquid dispensing advantageously provides selective control of liquid delivery. In combined drug delivery and diagnostic testing applications, such as in a system for delivering insulin and performing microdialysis blood glucose analysis, such delivery schemes have special significance; insulin may be stored in liquid storage volume **13** and dispensed independently while dialysate (such as saline solution) and a reagent are held in stored and dispensed from volumes **14** and **15,** preferably in a linked dispensing mode to be described.

Linked-dispensing commences when valves disposed in flow paths **8** and **9** are opened. Secondary piston **3** and its distal flange surface **21** advance in unison in housing **1** thereby expelling liquids simultaneously from storage volumes **14** and **15.** Storage volumes **14** and **15** are the same length to ensure that secondary piston 3 and its flange **16** fill each storage volume entirely when they reach a fully displaced position at the end of their range of movement. Given the constant length of volumes **14** and **15,** the cross-sectional area of each of each storage volume defines the amount of liquid expelled as piston **3** advances. Accordingly, the ratio of the cross-sectional surfaces of storage volumes **14** and **15** defines the volumetric ratio at which the liquids are dispensed. In the above mentioned combined insulin administration and microdialysis unit, this feature again has special significance because saline solution and reagent, stored in either of storage volumes **14** and **15,** are dispensed in the required, fixed volumetric ratio. In some cases, control of both flow rates may be achieved by controlling a valve in only one of the outlet flow paths while the other remains continuously open. Since liquid is only released from storage volumes **14** and **15** in fixed volumetric ratio, neither liquid flows unless both flow paths are open. It should be appreciated that embodiments in which linked dispensing precedes independent dispensing or both dispensing schemes are performed simultaneously are included within the scope of the present invention. Replacement liquid is injected into primary liquid storage volumes **13** and **13a** through septum **23** and similarly, additional replacement liquid is injected into liquid storage volume **15** through septum **25.** However, given that storage volumes **14** is non-contiguous with housing **1,** replacement liquid is introduced by way of an extended needle, or similar instrument, capable of spanning the entire length of storage-volume **13** and piecing septum **24.** Additional issues regarding the refilling of the storage reservoirs will be discussed later in the document.

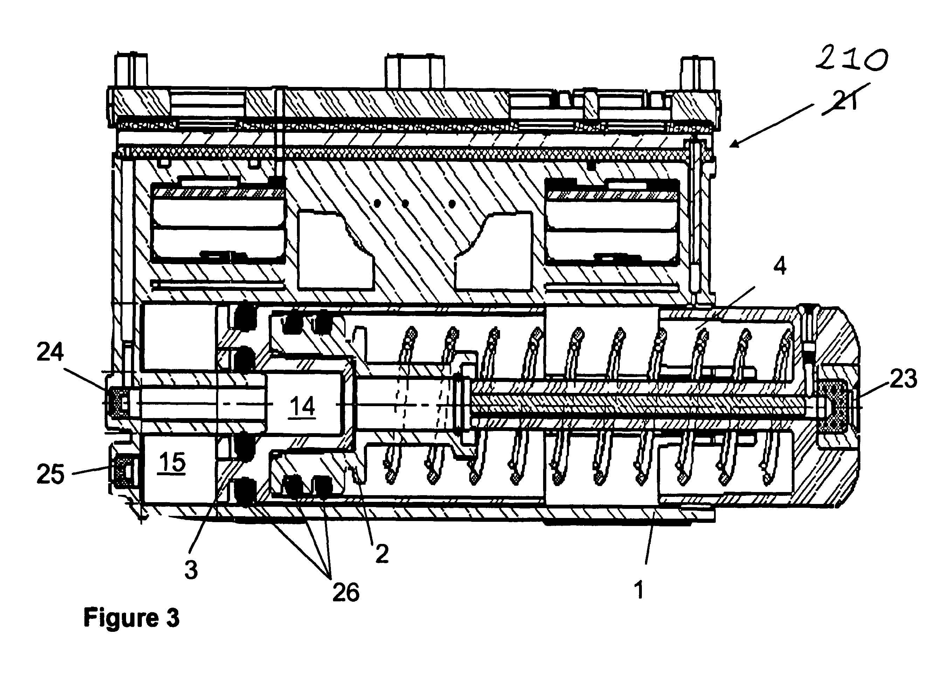

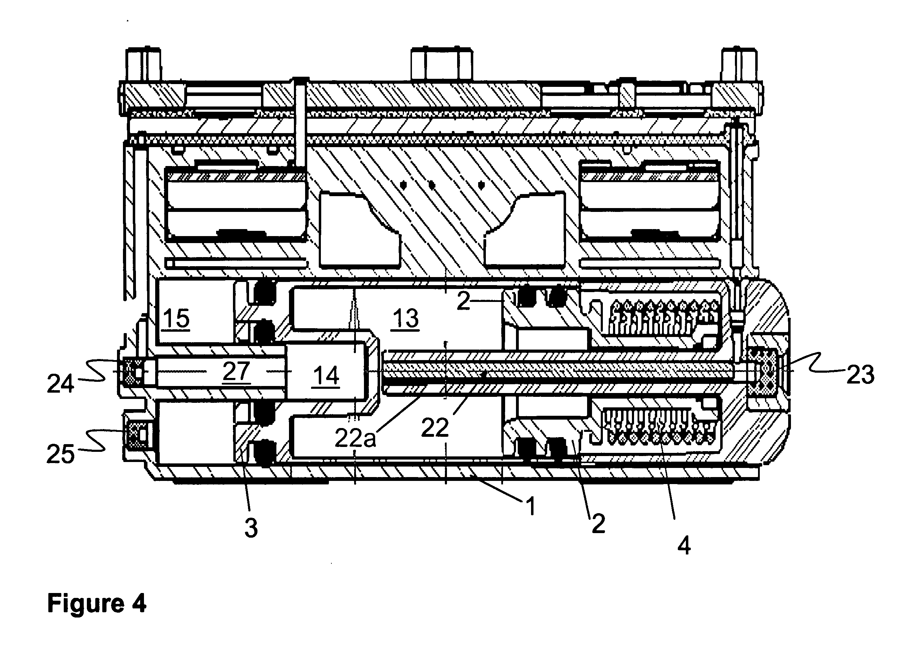

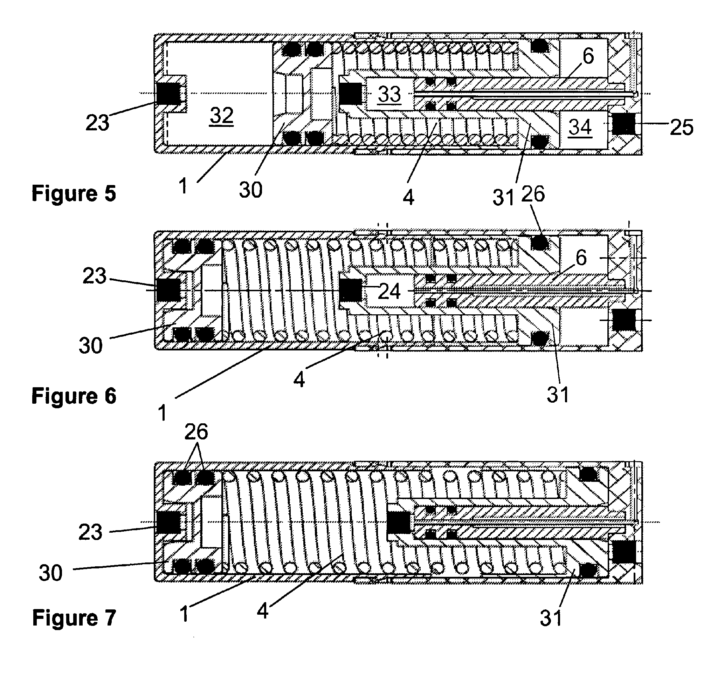

Figures 3 and 4 depict a non-limiting, alternative embodiment including an associated valve control arrangement 210. Generally speaking, the reservoir system of this embodiment is similar to that of Figures 1 and 2, and equivalent elements are designated similarly. Any suitable control system 210 may be used. PCT publication WO 2004/105827 discloses a non-limiting example of an appropriate control system; Figures 6-14 of the aforementioned PCT publication and their associated description are hereby incorporated by reference herein. Figure 3 further depicts biasing element in a non-loaded state. It should be appreciated that biasing element **4** may be implemented as any suitable biasing element. Exemplary embodiments illustrated here employ helical, compression springs; however, helical tension springs or non-helical springs constructed from metallic or non-metallic materials are included within the scope of the present invention. Furthermore, it should be noted that air springs or compressed gas equivalents are also included within the scope of the present invention.

Figure 4 depicts the above-mentioned alternative embodiment in a spring loaded, filled state prior to dispensing. A flow path **22** is disposed in a flow conduit **22a** projecting through the space circumscribed by spring **4,** an opening in primary piston **2,** and through the majority of primary liquid storage volume **13.** Liquids in primary storage volume **13** are expelled through a flow path inlet disposed at the distal end of storage volume **13.** Primary piston **2** is in leak free, sliding engagement with flow path **22.**

The storage reservoirs of each embodiment are refilled with the appropriate liquids by way of their flow paths and/or by suitably positioned septum seals. For example, liquid is injected though septum **23** into flow path **22** and into primary liquid storage volume **13.** Similarly, the appropriate liquids are injected through septum **25** directly into liquid storage volume **15** and through septum **24** into flow path **27** leading into liquid storage volumes **14.** It should be noted that, when refilling storage volumes involved in linked dispensing, both liquids most be replenished simultaneously to ensure that each volume is entirely filled. A void remaining in either of the storage volumes will distort the fixed volumetric ratio at which the liquids are to be delivered. Therefore, each liquid must be injected into the relevant storage volumes simultaneously at flow rates corresponding to the volumetric flow ratio defined by the structure, as discussed above. In practical terms, one replacement liquid is preferably injected into a first storage volume while the remaining flow path is in sealed, air tight connection with the second replacement liquid. The injected replacement liquid fills the storage volume, pushing the piston and its associated flange in unison thereby creating a partial vacuum that draws in the second liquid as the piston retracts.

Figures 5-7 depict a third, structurally analogous, embodiment having resilient biasing spring **4** disposed in between axially aligned rearward piston **30** and forward piston **31,** biasing each piston towards its corresponding end of the housing. Rearward piston **30** partially defines liquid storage volume **32** that is not in fluid communication with liquid storage volumes **33** and **34.** As rearward piston **30** advances in a rearward direction (to the left as shown), liquid held in storage volume **32** is dispensed independently of the liquids held in storage volumes **32** and **33.** Figure 6 shows the state of the device after the liquid from volume **32** is completely dispensed. Forward piston **31** partially defines volumes **32** and **33** also not in fluid communication with each other, so that as forward piston **31** moves in a forward direction (to the right as shown), liquids held in both storage volumes **32** and **33** are dispensed simultaneously in a fixed, volumetric ratio as described above. Figure 7 shows the empty device after all liquids have been dispensed.

It will be appreciated that the present invention is of particular advantage in any application where two or more liquids must be stored in a compact volume and be supplied under pressure. As mentioned above, the present reservoir system has particular application in regards to a combined Continuous Glucose Measurement (CGM) and Continuous Subcutaneous Insulin Infusion (CSII) system. However, it should be noted that the system has application in the administration of insulin with glucagon or GLP (Glucagon-Like Peptide), or insulin with other drugs and enzymes, and also for independent delivery of liquids and fluid mixing.

The reservoir system and its various components may be constructed from any suitable materials including, but not limited to, polymeric materials and metallic materials as is known in the art.

It will be appreciated that the above descriptions are intended only to serve as examples, and that many other embodiments are possible within the scope of the present invention as defined in the appended claims.

## Claims

1. A pressurized reservoir system for storing and dispensing liquids comprising;
(a) a housing (1);
(b) a piston arrangement in said housing including at least one piston (2, 3, 30, 31), said piston arrangement at least partially defining at least two liquid-storage volumes (13, 14, 15, 32, 33, 34); and
(c) a resilient biasing element (4) configured to bias said piston arrangement to pressurize both of said first and second liquid-storage volumes,
**characterized in that** said at least two liquid-storage volumes (13, 14, 15, 32, 33, 34) are not in fluid communication with each other,
and **in that** said piston arrangement is configured to allow independent dispensing of each of the liquids stored in said liquid-storage volumes,
and **in that** the pressurized reservoir system further comprises:
(d) a first flow path (8) for dispensing a first stored liquid from a first of said liquid-storage volumes (14, 33); and
(e) a second flow path (7) for dispensing a second stored liquid from a second of said liquid-storage volumes (13, 34).

2. The pressurized reservoir system of claim 1, wherein said piston arrangement comprises two pistons (30, 31) in axial alignment, said biasing element (4) being disposed between said two pistons (30, 31) so as to bias both of said two pistons in opposite directions.

3. The pressurized reservoir system of claim 1, wherein said piston arrangement further comprises at least one floating piston (3).

4. The pressurized reservoir system of claim 1, wherein said piston arrangement comprises at least one piston (3, 31) having a cavity of parallel walls, wherein said parallel walls slidingly engage an extended body (6) thereby at least partially defining the first of said two liquid-storage volumes (14, 33).

5. The pressurized reservoir system of claim 4, wherein said at least one piston (3, 31) has a flange (21) extending from said parallel walls so as to be in sliding engagement with said housing (1) thereby partially defining the second of said two liquid-storage volumes (13, 34).

6. The pressurized reservoir system of claim 4, wherein said at least one piston is implemented as a floating piston (3).

7. The pressurized reservoir system of claim 5, wherein said extended body includes a static projection (6) from said housing.

8. The pressurized reservoir system of claim 5, wherein said, wherein said static projection (6) includes the flow path (8) of said first liquid-storage volume (14, 33).

9. The pressurized reservoir system of claim 1, wherein said housing (1) comprises a cylindrical wall.

10. The pressurized reservoir system of claim 1, wherein said biasing element (4) comprises a spring.

11. The pressurized reservoir system of claim 1, wherein said biasing element (4) comprises a compressed gas.

12. The pressurized reservoir system of claim 1, further comprising a valve-actuator control-system (210) configured for controlling valves regulating flow in said flow paths.

13. The pressurized reservoir system of claim 1, further comprising an outlet conduit at least partially containing one of said flow paths, said outlet conduit passing through an opening in said primary piston and extending into one of said liquid-storage volumes, said primary piston being in sealed sliding engagement with said outlet conduit.

## Patentansprüche

1. Unter Druck stehendes Reservoirsystem für das Speichern und Abgeben von Flüssigkeiten, das aufweist:
(a) ein Gehäuse (1);
(b) eine Kolbenanordnung im Gehäuse, die mindestens einen Kolben (2, 3, 30, 31) umfasst, wobei die Kolbenanordnung mindestens teilweise mindestens zwei Flüssigkeitsspeichervolumen (13, 14, 15, 32, 33, 34) definiert; und
(c) ein elastisches Vorspannelement (4), das ausgebildet ist, um die Kolbenanordnung vorzuspannen, um sowohl das erste als auch das zweite Flüssigkeitsspeichervolumen unter Druck zu setzen,
**dadurch gekennzeichnet, dass** die mindestens zwei Flüssigkeitsspeichervolumen (13, 14, 15, 32, 33, 34) nicht in Fluidverbindung miteinander sind,
und dadurch, dass die Kolbenanordnung ausgebildet ist, um ein unabhängiges Abgeben einer jeden der Flüssigkeiten zu gestatten, die in den Flüssigkeitsspeichervolumen gespeichert werden,
und dadurch, dass das unter Druck stehende Reservoirsystem außerdem aufweist:
(d) einen ersten Strömungsweg (8) für das Abgeben einer ersten gespeicherten Flüssigkeit aus einem ersten der Flüssigkeitsspeichervolumen (14, 33); und
(e) einen zweiten Strömungsweg (7) für das Abgeben einer zweiten gespeicherten Flüssigkeit aus einem zweiten der Flüssigkeitsspeichervolumen (13, 34).

2. Unter Druck stehendes Reservoirsystem nach Anspruch 1, bei dem die Kolbenanordnung zwei Kolben (30, 31) in axialer Ausrichtung aufweist, wobei das Vorspannelement (4) zwischen den zwei Kolben (30, 31) angeordnet ist, um so beide der zwei Kolben in entgegengesetzten Richtungen vorzuspannen.

3. Unter Druck stehendes Reservoirsystem nach Anspruch 1, bei dem die Kolbenanordnung außerdem mindestens einen schwimmenden Kolben (3) aufweist.

4. Unter Druck stehendes Reservoirsystem nach Anspruch 1, bei dem die Kolbenanordnung mindestens einen Kolben (3, 31) aufweist, der einen Hohlraum aus parallelen Wänden aufweist, wobei die parallelen Wände verschiebbar mit einem ausgezogenen Körper (6) in Eingriff kommen, wodurch mindestens teilweise das erste der zwei Flüssigkeitsspeichervolumen (14, 33) definiert wird.

5. Unter Druck stehendes Reservoirsystem nach Anspruch 4, bei dem der mindestens eine Kolben (3, 31) einen Flansch (21) aufweist, der sich von den parallelen Wänden aus erstreckt, um so in einem gleitenden Eingriff mit dem Gehäuse (1) zu sein, wodurch teilweise das zweite der zwei Flüssigkeitsspeichervolumen (13, 34) definiert wird.

6. Unter Druck stehendes Reservoirsystem nach Anspruch 4, bei dem der mindestens eine Kolben als ein schwimmender Kolben (3) ausgeführt ist.

7. Unter Druck stehendes Reservoirsystem nach Anspruch 5, bei dem der ausgezogene Körper einen statischen Vorsprung (6) aus dem Gehäuse umfasst.

8. Unter Druck stehendes Reservoirsystem nach Anspruch 5, bei dem der statische Vorsprung (6) einen Strömungsweg (8) des ersten Flüssigkeitsspeichervolumens (14, 33) umfasst.

9. Unter Druck stehendes Reservoirsystem nach Anspruch 1, bei dem das Gehäuse (1) eine zylindrische Wand aufweist.

10. Unter Druck stehendes Reservoirsystem nach Anspruch 1, bei dem das Vorspannelement (4) eine Feder aufweist.

11. Unter Druck stehendes Reservoirsystem nach Anspruch 1, bei dem das Vorspannelement (4) Druckgas aufweist.

12. Unter Druck stehendes Reservoirsystem nach Anspruch 1, das außerdem ein Ventil-Betätigungselement-Steuersystem (210) aufweist, das für das Steuern von Ventilen ausgebildet ist, die die Strömung in den Strömungswegen regulieren.

13. Unter Druck stehendes Reservoirsystem nach Anspruch 1, das außerdem eine Auslassleitung aufweist, die mindestens teilweise einen der Strömungswege enthält, wobei die Auslassleitung durch eine Öffnung im primären Kolben hindurchgeht und sich in eines der Flüssigkeitsspeichervolumen erstreckt, wobei der primäre Kolben in einem abgedichteten gleitenden Eingriff mit der Auslassleitung ist.

## Revendications

1. Système de réservoir sous pression pour le stockage et la distribution de liquides, comprenant :
(a) un boîtier (1) ;
(b) un assemblage de piston dans ledit boîtier, englobant au moins un piston (2, 3, 30, 31), ledit assemblage de piston définissant au moins en partie au moins deux volumes de stockage de liquide (13, 14, 15, 32, 33, 34) ; et
(c) un élément élastique de sollicitation (4), configuré de sorte à solliciter ledit assemblage de piston pour mettre sous pression lesdits premier et deuxième volumes de stockage de liquide ;
**caractérisé en ce que** lesdits au moins deux volumes de stockage de liquide (13, 14, 15, 32, 33, 34) ne sont pas en communication de fluide l'un avec l'autre ;
et **en ce que** ledit assemblage de piston est configuré de sorte à permettre une distribution indépendante de chacun des liquides stockés dans lesdits volumes de stockage de liquide
et **en ce que** le système de réservoir sous pression comprend en outre :
(d) une première trajectoire d'écoulement (8), pour distribuer un premier liquide stocké à partir d'un premier desdits volumes de stockage de liquide (14, 33) ; et
(e) une deuxième trajectoire d'écoulement (7), pour distribuer un deuxième liquide stocké à partir d'un deuxième volume desdits volumes de stockage de liquide (13, 34).

2. Système de réservoir sous pression selon la revendication 1, dans lequel ledit assemblage de piston comprend deux pistons (30, 31) alignés axialement, ledit élément de sollicitation (4) étant agencé entre lesdits deux pistons (30, 31) de sorte à solliciter lesdits deux pistons dans des directions opposées.

3. Système de réservoir sous pression selon la revendication 1, dans lequel ledit assemblage de piston comprend en outre au moins un piston flottant (3).

4. Système de réservoir sous pression selon la revendication 1, dans lequel ledit assemblage de piston comprend au moins un piston (3, 31) comportant une cavité de parois parallèles, lesdites parois parallèles s'engageant de manière coulissante dans un corps étendu (6), définissant ainsi au moins en partie le premier desdits deux volumes de stockage de liquide (14, 33).

5. Système de réservoir sous pression selon la revendication 4, dans lequel ledit au moins un piston (3, 31) comporte une bride (21) s'étendant à partir desdites parois parallèles en vue d'un engagement coulissant dans ledit boîtier (1), définissant ainsi en partie le deuxième desdits deux volumes de stockage de liquide (13, 34).

6. Système de réservoir sous pression selon la revendication 4, dans lequel ledit au moins un piston a la forme d'un piston flottant (3).

7. Système de réservoir sous pression selon la revendication 5, dans lequel ledit corps étendu englobe une saillie statique (6) débordant dudit boîtier.

8. Système de réservoir sous pression selon la revendication 5, dans lequel ladite saillie statique (6) englobe la trajectoire d'écoulement (8) dudit premier volume de stockage de liquide (14, 33).

9. Système de réservoir sous pression selon la revendication 1, dans lequel ledit boîtier (1) comprend une paroi cylindrique.

10. Système de réservoir sous pression selon la revendication 1, dans lequel ledit élément de sollicitation (4) comprend un ressort.

11. Système de réservoir sous pression selon la revendication 1, dans lequel ledit élément de sollicitation (4) comprend un gaz comprimé.

12. Système de réservoir sous pression selon la revendication 1, comprenant en outre un système de commande d'actionnement de soupapes (210), configuré de sorte à contrôler des soupapes assurant la régulation de l'écoulement dans lesdites trajectoires d'écoulement.

13. Système de réservoir sous pression selon la revendication 1, comprenant en outre un conduit de sortie, contenant au moins en partie une desdites trajectoires d'écoulement, ledit conduit de sortie passant à travers une ouverture dans ledit piston primaire et s'étendant dans l'un desdits volumes de stockage de liquide, ledit piston primaire étant engagé de manière coulissante et étanche dans ledit conduit de sortie.
